# EUROPEAN PATENT SPECIFICATION

(11) **EP 4 108 223 B1**
(45) Date of publication and mention of the grant of the patent: **09.07.2025**
(21) Application number: 22176318.8
(22) Date of filing: 31.05.2022
(51) Int. Cl.: A61J 7/00, G16H 20/10

(54) **MODULAR AND AUTOMATIC DISPENSER FOR MEDICATION AND DISPENSING METHOD**
MODULARER UND AUTOMATISCHER SPENDER FÜR MEDIKAMENTE UND VERFAHREN ZUR ABGABE
DISTRIBUTEUR MODULAIRE ET AUTOMATIQUE DE MÉDICAMENTS ET MÉTHODE DE DISTRIBUTION

(30) Priority: 23.06.2021 PT 2021117303
(43) Date of publication of application: 28.12.2022
(73) Proprietor: Instituto Politécnico De Bragança, 5300-253 Bragança (PT)
(72) Inventor: Alegre da Costa, Bruno António, P-5300-034 Bragança (PT); Pinheiro Nunes Pereira, Ana Isabel, P-5300-271 Bragança (PT); Ribeiro de Mesquita, Luís Manuel, P-5300-849 Bragança (PT); Sanches de Castro Lopes, Rui Pedro, P-5300-675 Bragança (PT); Sousa Magalhães Lima, José Luís, P-4500-706 Nogueira da Regedoura (PT); Ferro Lebres, Vera Alexandra, P-5300-274 Bragança (PT)
(74) Representative: Lourenço Martinho do Rosário, Ana Margarida

(56) References cited:
- US-A- 4 573 606
- US-A- 5 755 357

## Description

### Field of the invention

The present invention falls within the area of medicine, more particularly to an equipment for the automatic and timely dispensing of oral medicine, particularly in the form of pills, capsules or similar for delivery to at least one user.

### Scope of the invention

With the evolution of medicine and related sciences, human life expectancy is increasing, and the quality of life has also improved substantially. However, this increase in life expectancy, as well as its quality, is generally achieved by the periodic consumption of medicine, usually in the form of pills. As this is a situation that occurs with greater frequency in an elderly population, it is not uncommon that, due to the quantity and diversity of medicine that must be taken, and with the cognitive difficulties that some people develop, mistakes occur, with errors ranging from forgetting to take medicine, taking an inappropriate dosage, or taking at the wrong time. Serious situations can result from these occurrences, especially in cases where medicine is taken in the wrong dosage or if users self-medicate or take the wrong pills.

Additionally, difficulties remain in remote access to information by formal and/or informal caregivers. These caregivers must go to the home of the users to help them take their medicine. In case of unexpected situations, these caregivers are sometimes unaware of the history of previous occurrences that may have occurred.

### Background of the invention

Over the years, devices capable of storing and dispensing pills have been developed. With an alarm system, these devices alert users when it is time to take their medicine. However, these devices do not safeguard the preservation of medicine, the communication with the system, the modularity in storage, as well as an efficient and intelligent management.

The document US2006/0266763 presents an automatic pill dispenser, which has a microprocessor and some interesting features. However, this model does not allow connection to external servers. The model does not allow updating the medicine remotely. The storage of the pills is done in such a way that it does not allow changing the doses, and there is no flexibility for changes during normal operation, if needed. The pills are stored outside the blister packs, which can be a factor in pills contamination and reduced efficacy. There is also no efficient use of storage space for pills. It does not have protocols for emergency scenarios nor the possibility of communication with a smart wristband or other local devices.

The documents US2014/0097194 and US2014/0131378 show automatic pill dispensers with a mechanical arm that integrates an aspirator that takes the pill to be dispensed from the storage section and delivers it to the dispensing box.

The document US 2016/0042150 introduces a smart pill dispenser.

The document US10555873 discloses a modular pill dispenser system. This system does not allow communication with other devices, i.e., it does not provide bidirectional functionality, relevant for user authorization with the dispenser and for extending the alarm range of the dispenser. Medicine is sensitive and can be dangerous or harmful if unauthorized users have access to it. The model does not provide a way to verify the identity of the user. The dispenser alarm may not be noticed by the user.

The document US5755357A discloses an apparatus for dispensing medication. The apparatus includes a housing carrying first and second carousels, each of which is adapted to hold medication. However, the apparatus does not comprises the delivery cup presence sensors checks the removal of the delivery cup from the medicine dispenser; and a counting sensor; and a weight sensor and does not comprises a vibration motor which is activated which releases medicine units that may be stuck.

### Advantages of the invention

The invention is defined by the appended claims and consists of a modular, automatic, and intelligent device, with low production cost for entry into the international market. The medicine dispenser may contain a mechanical component, an electronic component and a computer component. The medicine dispenser is configured to store, organize and dispense prescribed medicine to the user or users of the equipment. The dispenser also allows coupling to other dispensers, which allows increasing the number of users that can benefit from the equipment, with the increase of the storage area.

### Brief description of the figures

These and other characteristics can be easily understood by means of the attached drawings, which are to be considered as mere examples and in no way restrictive of the scope of the invention. In the drawings, and for illustrative purposes, the measurements of some of the elements may be exaggerated and not drawn to scale. The absolute and relative dimensions do not correspond to the real ratios for the embodiments of the invention.

In a preferred embodiment:

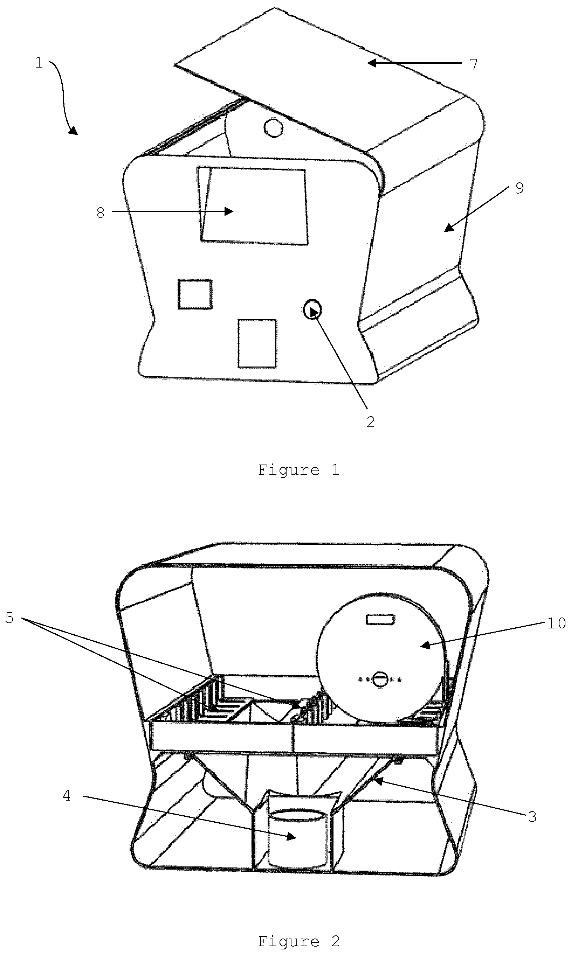

Figure 1 shows a front perspective view of the equipment of the invention.
Figure 2 shows the equipment of the invention in a frontal position and its interior.

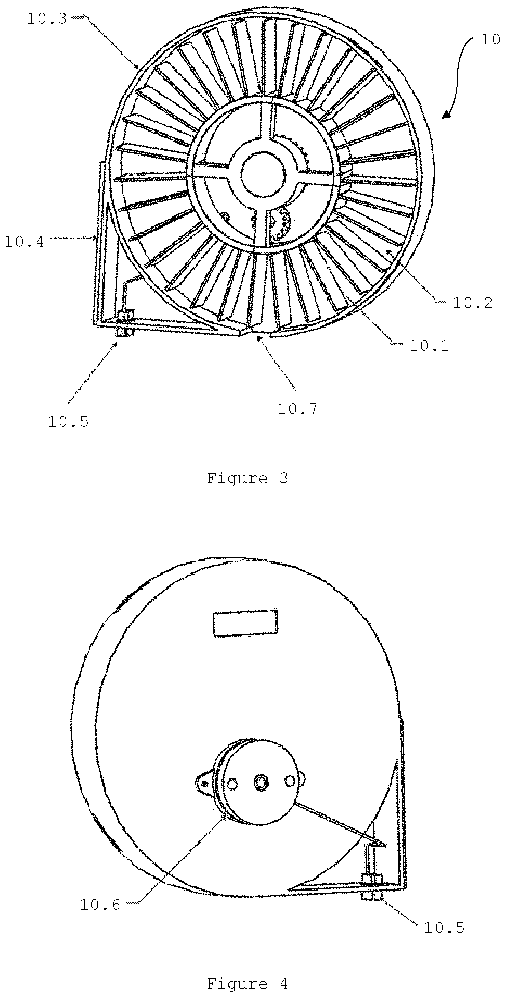

Figure 3 shows a front view of the dispenser module.
Figure 4 shows the back of the dispenser module.

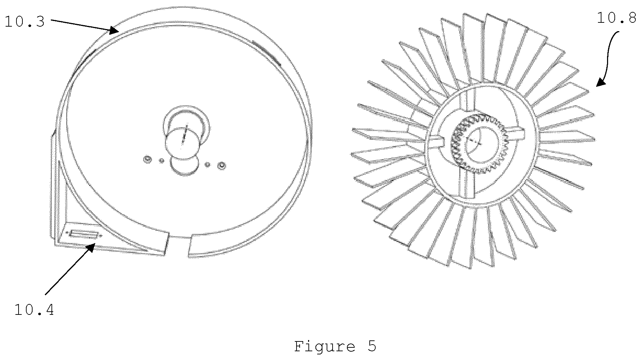

Figure 5 shows a detail of the components of the dispenser module.

In the figures are marked the elements and components of the equipment of the present invention, as well as the elements essential for the functioning of the invention:
1. medicine dispenser
2. button
3. router
4. delivery cup
5. housing
7. cover
8. operation panel
9. chassis
10. dispenser module
   10.1. partitions
   10.2. lockers
   10.3. wrapper
   10.4. guide
   10.5. connector
   10.6. rotation system
   10.7. opening
   10.8. storage divider

### Detailed description of the invention

By "substantially cylindrical" and "substantially trapezoidal" shapes is meant the preferred shapes for embodiments of the invention, although the invention is able to work with other shapes.

By "substantially vertical", "at the back" and "at the bottom" positions is meant the preferred positions for implementing the invention, although the invention is able to work with other positions.

By "medicine units" is meant that the quantity is at least one medicine unit.

According to the figures, the present invention relates to a modular and automatic dispenser for medicine, intended for the automatic and timely dispensing of medicine, particularly in the form of pills, capsules or similar for delivery to at least one user, comprising a storage system, a delivery system and a management and communication system.

The medicine dispenser (1) integrates a chassis (9) comprising:
- a cover (7) for access to the interior of the medicine dispenser (1);
- a multifunction button (2) located on the front of the chassis (9) which allows for, among other things, deactivating the alarm;
- an operation panel (8) for communication with the user and that may include buttons and a display for user interface which allows for, namely, but not exclusively, programming and activation of functionalities and uploading of user related information to the prescription and remote devices.

In the interior of the medicine dispenser (1) are located the storage system and the delivery system, which comprise the mechanical components for storing and delivering medicine, as well as the electronic components, namely the circuit board, which allow the instructions received from the management system, and which control the mechanical components, to be executed. The medicine dispenser (1) also has a mechanism for coupling to other medicine dispensers (1).

### Storage system

The storage system comprises:
- at least one removable dispenser module (10); and
- at least one housing (5) to which the at least one dispenser module (10) is fixed.

The housing (5), in a preferred embodiment, has substantially an "L" shape and incorporates means that allow the at least one dispenser module (10) to be attached. The lower wall of the housing (5) is placed in a substantially horizontal position and the side wall of the housing (5) is placed in a substantially vertical position. The lower wall of the housing (5) additionally comprises a connector (10.5). In a preferred embodiment, the means enabling the attachment of the dispenser module (10) are rails which are coupled to the bottom wall and the side wall of the housing (5). In another embodiment, the housing (5) has any shape suitable to fit the at least one dispenser module (10).

The dispenser module (10), in a preferred embodiment, has a substantially cylindrical shape, is placed in a substantially vertical position within the chassis (9) and integrates a wrapper (10.3) having a substantially circular shape, encircling a storage divider (10.8) and which comprises an opening (10.7) in its lower part through which the medicine are dispensed. The storage divider (10.8) integrates at least one partition (10.1) separating the at least one locker (10.2) used for storing medicine. Each locker (10.2) is substantially trapezoidal in shape, with a width that narrows from the outside to the inside, the inside being the wall in the centre of the storage divider (10.8). As the medicine, and consequently the casings protecting them, can be of different sizes and shapes, the size of the lockers (10.2) can be changed by adjusting the distance between partitions (10.1) by moving the partitions (10.1) on the centre wall of the storage divider (10.8), so that the lockers (10.2) can suit the size of the medicine or the casing of the medicine to be stored. As the wrapper (10.3) surrounds the storage divider (10.8), inappropriate outflow of medicine from the lockers (10.2) is prevented. The dispenser module (10) additionally comprises means enabling the at least one dispenser module (10) to be fixed to the housing (5). In a preferred embodiment, the means for attaching the dispenser module (10) to the housing (5) is a guide (10.4) which is shaped to fit into the existing rails of the housing (5). In one embodiment, the guide (10.4) and the housing (5) have mutually complementary shapes. In a preferred embodiment, the guide (10.4) has substantially an "L" shape, whereby both walls of the guide (10.4), i.e. the side wall and the bottom wall, have a length substantially equal to the radius of the wrapper (10.3), whereby the bottom wall of the guide (10.4) engages in rails of the bottom wall of the housing (5) and the side wall of the guide (10.4) engages in rails of the side wall of the housing (5).

The dispenser module (10) also incorporates a connector (10.5) for connection between the management and communication system and the rotation system (10.6) of the dispenser module (10). Located at the rear of the dispenser module (10), the rotation system (10.6) integrates the rotation components of the storage divider (10.8).

The rails of the housing (5) are configured to receive the guides (10.4) of the dispenser module and comprise guide fixing mechanisms (10.4) that allow the dispenser module (10) to be coupled and uncoupled. The rails of the housing (5) also comprise mechanisms for joining the electronics to the connector (10.5) of the dispenser module (10). Due to the shape and position of the guide (10.4), the dispenser module (10) is fixed to the housing (5) in a substantially vertical position.

The dispenser module (10) can be easily removed and inserted in the interior of the medicine dispenser (1), facilitating the refilling of the dispenser module (10) whenever necessary. After gaining access to the housing (5), through the opening of the cover (7), the guides (10.4) allow for easy insertion and removal of the dispenser modules (10) from the interior of the medicine dispenser (1).

### Delivery system

The medicine delivery system allows for the collection of medicine and its routing to the outlet.

The delivery system comprises a medicine router (3), positioned under the dispenser module (10), comprising:
- a counting sensor selected from, namely, but not exclusively, an infrared sensor, a microwave sensor or an ultrasonic sensor;
- a vibration motor;
- a weight sensor;
- a delivery cup presence sensor (4); and
- a delivery cup (4).

The router (3) presents any shape suitable for routing the medicine that falls into it, transferring said medicine, by gravity, to the delivery cup (4) positioned under the router (3). In a preferred embodiment, the router (3) has a semifunnel shape.

The counting sensor checks if the medicine units have left the dispensing module (10). The weight sensor ascertains if the medicine units have been delivered to the delivery cup (4) and the presence sensor of the delivery cup (4) confirms if the delivery cup (4) has already been removed or is still in the medicine dispenser (1). After the medicine unit is released from the dispenser module (10), the counting sensor confirms that the amount of medicine units that fell into the router (3) corresponds to the number of medicine units that had to fall by sensing the passage of the medicine units to the router (3). If the counting sensor does not detect the passage of the medicine units, the vibration motor is activated which releases medicine units that may be stuck. The weight sensor checks if the medicine units have been deposited in the delivery cup (4), by making a comparison between the weight of the medicine units inside the delivery cup (4) and the weight of the medicine units that should be inside the delivery cup (4). The presence sensor of the delivery cup (4) checks whether the delivery cup (4) has already been removed from the medicine dispenser (1).

### Management and communication system

Additionally, the medicine dispenser (1) comprises a management and communication system.

The management and communication system has the function of managing the medicine dispenser (1), as well as the communication between the circuit board of the medicine dispenser (1), the servers and the databases comprising the information necessary for the operation of the medicine dispenser (1). These servers and databases namely comprise the data regarding the user, the prescription, the medicine, where it is stored in the dispenser modules (10) and the respective dispensing schedules.

The communication of the medicine dispenser (1) with the server, the databases and the application, that can run on a fixed or mobile electronic device, namely, but not exclusively, tablet, smartphone, laptop, or desktop, can be performed through various channels that can be activated and deactivated according to needs. The communication channels are selected from, namely, but not exclusively, Ethernet, Wi-Fi, Bluetooth, Radio Frequency Identification (RFID), Near Field Communication (NFC), Global System for Mobile Communication (GMS), Zigbee, 3G, 4G, 5G, Microwave, and Long Range (LoRa). This redundancy of channels allows adaptation to various scenarios and the choice of channel according to the situation.

The communication carried out between the server and the application allows, in particular:
- registration and update of users;
- recording and update of the actions to be taken in the event of the occurrence of a non-conformity;
- recording and update of prescription information;
- sending log records relating to the operation of the medicine dispenser (1);
- sending of alerts and notifications; and
- software updates.

The communication system also allows communication with remote devices, including, but not limited to, smart bracelets, smartwatches, RFID/NFC tags, or smartphones. These devices allow, namely, the authentication of the user at the medicine dispenser (1) and the update of information regarding the schedule for taking medicine. The communication channels for authentication are selected from but not exclusively, Bluetooth, Bluetooth Low Energy, NFC and RFID.

Additionally, the management and communication system comprises an alarm that is triggered when it is time to take the medicine, to inform of the dispensing of medicine. This alarm can be triggered on the medicine dispenser (1) through a visual/sound component that integrates the medicine dispenser (1), as well as on the remote devices.

### Method of operation

Since the medicine dispenser (1) is an equipment that can be used by several users, when used for the first time by a user, it is necessary to insert information about the user and the respective prescription. This information can be loaded locally through the operation panel (8), through the application installed in fixed or mobile devices, or by connecting to external servers that have the information previously loaded.

Regarding the prescription, it is necessary to load the information regarding the medicine and its location within the medicine dispenser (1), i.e., which dispenser module (10) the medicine will stored in, and the respective dispensing schedules. All medicine are placed in the respective lockers (10.2) through the opening of the lid (7) that allows access to the inside of the medicine dispenser (1) .

For a first stage, the parameterization stage, it is necessary to proceed with the activation of the intended functionalities, as well as the specification of the operating characteristics. One of the functionalities to be activated is the need for user authentication. Another of the existing functionalities, whose operating characteristics need to be defined, are the alarm parameters. For example, what type of alarm is activated, whether it is visual, acoustic or both visual and acoustic, whether it is activated locally and/or through remote devices and for how long it is active. It is also necessary to inform the medicine dispenser (1) of what action to take if the alarm is not deactivated within the time set. If there is more than one user, each user defines their own parameters. The parameters will be saved and used in the interaction with the respective user.

During the parameterization of a user, it is also possible to register remote devices associated with the user, namely, but not exclusively, smart wristbands, smartwatches, RFID/NFC tags, or smartphones. Each user can have more than one remote device associated, however, a given remote device can only be associated with one user.

For a second step, the delivery step, when the time comes to dispense a medicine according to the prescription time, the alarm of the medicine dispenser (1) is activated.

The user or users, or someone duly authorized to do so, namely a formal and/or informal caregiver, comes or goes to the medicine dispenser (1) at the time set in the timetable to collect the medicine to be taken.

If authentication is active, the alarm is deactivated, and the medicine is dispensed by user authentication with the medicine dispenser (1). This authentication is executed by entering a code on the operation panel (8), through a remote device or through an application installed on a mobile device. An alarm is always related to a medicine prescription for one user. The device uses the parameters previously entered to alert the user to take the dose. Authentication is only authorized if the code is correctly entered in the operation panel (8), by the remote device or by the application installed on a mobile device associated to the user who must take the dose.

If the user authentication option is not active, pressing the multifunction button (2) deactivates the alarm and the medicine is dispensed.

If the alarm is not deactivated within the time set for this, the action configured in the medicine dispenser (1) is triggered. Before dispensing, the device informs the user to whom the medicine to be dispensed belongs to via the operating panel (8) or an acoustic signal.

Once the medicine dispenser (1) has confirmed that the user is near the equipment, the process of delivering and collecting the medicine begins.

After the alarm is deactivated, the management and communication system send information about the location of the medicine to be dispensed and the quantity to be dispensed to the circuit board that controls the storage system and the delivery system. Upon receiving this information, the circuit board activates the rotation system (10.6) of the dispensing module (10) in which the medicine to be dispensed is located, sets the storage divider (10.8) in rotation, until the locker (10.2) containing the medicine units to be dispensed is substantially aligned with the opening (10.7) of the wrapper (10.3). Since the opening (10.7) is located at the bottom of the wrapper (10.3) of the dispensing module (10), when the locker (10.2) becomes substantially aligned with the opening (10.7), through the force of gravity the amount of medicine units to be dispensed falls into the router (3). The counting sensor detects the passage of the medicine units into the router (3), which lets the management and communication system know that the medicine units have left the locker (10.2).

Given its inclination of the router (3), the drug units are routed into the delivery cup (4) by the router (3). The weight sensor located underneath the delivery cup (4) checks whether the units of medicine deposited in the delivery cup (4) match the number of units of medicine that should be delivered, by making a comparison between the weight of the units of medicine deposited in the delivery cup (4) and the weight of the units of medicine that should be inside the delivery cup (4). With the medicine dispensed, the user collects the delivery cup (4) with the units of medicine. Finally, the delivery cup presence sensor checks whether the delivery cup (4) has been removed from the medicine dispenser (1).

If the management system detects any non-conformity in any of the previous situations, namely when dropping the medicine units from the locker (10.2), delivering the medicine units to the delivery cup (4) or collecting the delivery cup (4), the management system shall activate the emergency measures foreseen for each situation, namely sending messages, activating the acoustic warning, light warning, or any other that is appropriate for the gravity of the situation.

## Claims

1. Modular and automatic dispenser for medicine comprising:
- a chassis (9);
- a storage system comprising:
- at least one removable dispenser module (10) comprising a wrapper (10.3) encircling a storage divider (10.8) and comprising an opening (10.7) at the bottom; and
- a housing (5);
- a delivery system which comprises a medicine router (3) positioned under the dispensing module (10) and a delivery cup (4) placed under the router (3);
- the dispenser module (10) comprises a guide (10.4) located at the bottom of the dispenser module (10);
- the housing (5):
- has a format substantially complementary to the format of the guides (10.4);
- comprises rails configured to receive the guides (10.4); and
- comprises guide (10.4) fixing mechanisms that allow coupling and uncoupling of the dispenser module (10);
- the dispenser module (10) is fixed in the housing (5) in a substantially vertical position;
**characterised in that**:
- the dispenser module (10) comprises:
- a counting sensor which checks if the medicine units have left the dispensing module (10);
- a weight sensor which ascertains if the medicine units have been delivered to a delivery cup (4);
- a delivery cup presence sensor which confirms if the delivery cup (4) has already been removed or is still in the medicine dispenser (1); and a vibration motor which is activated if the counting sensor does not detect the passage of the medicine units.

2. Dispenser according to the preceding claim, **wherein** the counting sensor is selected from an infrared sensor, a microwave sensor or an ultrasonic sensor.

3. Dispenser according to any one of the preceding claims, **wherein** the guide (10.4) having an "L" shape, wherein the lower wall of the guide (10.4) fits into the trough of the lower wall of the housing (5) and the side wall of the guide (10.4) fits into the side wall of the housing (5) and said both walls have a length substantially equal to the radius of the wrapper (10.3).

4. Dispenser according to any one of the preceding claims, **wherein** the dispenser module (10) comprises:
- a rotation system (10.6) comprising the rotation components of the storage divider (10.8);
- a connector (10.5) connecting the management and communication system to the rotation system (10.6);
and the storage divider (10.8) comprising at least one partition (10.1) which separates a at least one locker (10.2).

5. Dispenser according to the preceding claim, **wherein** the locker (10.2) has a substantially trapezoidal shape with a width that narrows from the outside to the inside, the inside being the wall of the center of the storage divider (10.8).

6. Dispenser according to the preceding claim, **wherein** the size of the locker (10.2) is changed by adjusting the distance between partitions (10.1) by moving the partition (10.1) on the wall in the centre of the storage divider (10.8).

7. Dispenser according to any one of the preceding claims, **wherein** the dispenser module (10) has a substantially cylindrical shape.

8. Dispenser according to any of the preceding claims, **wherein** the wrapper (10.3) has a substantially circular shape.

9. Dispenser according to any one of the preceding claims, **wherein** the chassis (9) comprises a cover (7), a multifunction button (2) and an operations panel (8) for inserting. the specification of the operating characteristics, namely the information on the user and the respective prescription, the user authentication functionality, the alarm parameters, the actions to be triggered in the event of the occurrence of a non-conformity.

10. Dispenser according to any one of the preceding claims, **wherein** it further comprises an electronic component comprising a circuit board, a sensor system and a management and communication system enabling electronic and computer management of the dispenser.

11. Dispenser according to the preceding claim, wherein the communication of the medicine dispenser (1) with servers and databases is configured to run on a fixed or mobile electronic device.

12. Method of dispensing medicines with the dispenser according to claims 9-11 **characterized by** comprising the following steps:
a. parameterization stage, comprising the following steps:
a.1 insertion of information on the user and the respective prescription;
a.2 activation of the user authentication functionality;
a.3 setting of alarm parameters;
a.4 defining the actions to be triggered in the event of the occurrence of a non-conformity;
b. delivery stage, comprising the following steps:
b.1 according to the prescription time, the alarm is activated;
b.2 the alarm is deactivated by authentication of the user at the medicine dispenser (1) or by pressing the multifunction button (2);
b.3 after the alarm is deactivated, the rotation system (10.6) of the dispenser module (10) is activated by the circuit board;
b.4 with the rotation of the storage divider (10.8), the locker (10.2) containing the medicine units to be dispensed becomes substantially aligned with the opening (10.7) of the wrapper (10.3);
b.5 the amount of medicine units to be dispensed falls to the router (3);
b.6 the counting sensor detects the passage of the medicine units to the router (3);
b.7 the medicine units are forwarded to the delivery cup (4) by the router (3);
b.8 the weight sensor checks whether the weight of the medicine units deposited in the delivery cup (4) corresponds to the weight of the medicine units that should be inside the delivery cup (4);
b.9 a user picks up the delivery cup (4); and
b.10 the delivery cup presence sensor checks the removal of the delivery cup (4) from the medicine dispenser (1).

## Patentansprüche

1. Modularer und automatischer Medikamentenspender, umfassend:
- ein Chassis (9);
- ein Aufbewahrungssystem, umfassend:
- mindestens ein herausnehmbares Spendermodul (10), umfassend eine Hülle (10.3), die eine Teilereinheit (10.8) umgibt, wobei die Hülle (10.3) eine Öffnung (10.7) an ihrer Unterseite aufweist; und
- ein Gehäuse (5);
- ein Ausgabesystem, umfassend einen unterhalb des Spendermoduls (10) angeordneten Medikamenten-Verteiler (3) sowie einen unterhalb des Medikamenten-Verteilers (3) angeordneten Ausgabebecher (4);
- wobei das Spendermodul (10) an seinem unteren Ende eine Führung (10.4) aufweist;
- wobei das Gehäuse (5):
- eine im Wesentlichen komplementäre Form zu derjenigen der Führungen (10.4) aufweist;
- Schienen umfasst, die zum Aufnehmen der Führungen (10.4) ausgelegt sind; und
- Befestigungsmechanismen für die Führungen (10.4) umfasst, die ein Kuppeln und Entkuppeln des Spendermoduls (10) ermöglichen;
- wobei das Spendermodul (10) in einer im Wesentlichen vertikalen Position im Gehäuse (5) fixiert ist;
**dadurch gekennzeichnet, dass:**
- das Spendermodul (10) Folgendes umfasst:
- einen Zählsensor, der überprüft, ob die Medikationseinheiten das Spendermodul (10) verlassen haben;
- einen Gewichtssensor, der feststellt, ob die Medikationseinheiten in den Ausgabebecher (4) abgegeben wurden;
- einen Ausgabebecher-Präsenzsensor, der bestätigt, ob der Ausgabebecher (4) bereits entnommen wurde oder sich noch im Medikamentenspender (1) befindet; und
- einen Vibrationsmotor, der aktiviert wird, wenn der Zählsensor keinen Durchgang von Medikationseinheiten registriert.

2. Medikamentenspender nach Anspruch 1, **wobei** der Zählsensor ein Infrarotsensor, ein Mikrowellensensor oder ein Ultraschallsensor ist.

3. Medikamentenspender nach einem der vorhergehenden Ansprüche, **wobei** die Führung (10.4) eine L-Form aufweist, wobei die untere Wand der Führung (10.4) in die Rinne der unteren Wand des Gehäuses (5) und die Seitenwand der Führung (10.4) in die Seitenwand des Gehäuses (5) eingreift, wobei beide Wände eine Länge aufweisen, die im Wesentlichen dem Radius der Hülle (10.3) entspricht.

4. Medikamentenspender nach einem der vorhergehenden Ansprüche, **wobei** das Spendermodul (10) Folgendes umfasst:
- ein Rotationssystem (10.6) mit Rotationskomponenten für die Teilereinheit (10.8);
- einen Verbinder (10.5), der das Management- und Kommunikationssystem mit dem Rotationssystem (10.6) verbindet;
wobei die Teilereinheit (10.8) mindestens eine Trennwand (10.1) aufweist, die mindestens ein Fach (10.2) voneinander abgrenzt.

5. Medikamentenspender nach dem vorhergehenden Anspruch, **wobei** das Fach (10.2) eine im Wesentlichen trapezförmige Form aufweist, deren Breite sich von außen nach innen verjüngt, wobei die Innenseite die Wand im Zentrum der Teilereinheit (10.8) ist.

6. Medikamentenspender nach dem vorhergehenden Anspruch, **wobei** die Größe des Fachs (10.2) durch Verstellen des Abstands zwischen den Trennwänden (10.1) verändert werden kann, indem die Trennwand (10.1) auf der Wand im Zentrum der Teilereinheit (10.8) verschoben wird.

7. Medikamentenspender nach einem der vorhergehenden Ansprüche, **wobei** das Spendermodul (10) eine im Wesentlichen zylindrische Form aufweist.

8. Medikamentenspender nach einem der vorhergehenden Ansprüche, **wobei** die Hülle (10.3) eine im Wesentlichen kreisförmige Form aufweist.

9. Medikamentenspender nach einem der vorhergehenden Ansprüche, **wobei** das Chassis (9) eine Abdeckung (7), eine Multifunktionstaste (2) und ein Bedienfeld (8) umfasst, über welches Betriebseigenschaften wie Benutzerinformationen und jeweilige Verschreibung, Benutzer-Authentifizierungsfunktion, Alarmparameter sowie auszulösende Maßnahmen bei Auftreten einer Nichtkonformität eingegeben werden.

10. Medikamentenspender nach einem der vorhergehenden Ansprüche, **wobei** er ferner eine elektronische Komponente umfasst, bestehend aus einer Leiterplatte, einem Sensorsystem sowie einem Management- und Kommunikationssystem, welches die elektronische und rechnergestützte Verwaltung des Spenders ermöglicht.

11. Medikamentenspender nach dem vorhergehenden Anspruch, **wobei** die Kommunikation des Medikamentenspenders (1) mit Servern und Datenbanken über ein festes oder mobiles elektronisches Gerät erfolgt.

12. Verfahren zum Ausgeben von Medikamenten mittels des Medikamentenspenders gemäß den Ansprüchen 9 bis 11, **dadurch gekennzeichnet, dass** es die folgenden Schritte umfasst:
a. Parametrierungsphase, umfassend die folgenden Schritte:
a.1 Eingabe der Benutzerinformationen und der jeweiligen Verschreibung;
a.2 Aktivierung der Benutzer-Authentifizierungsfunktion;
a.3 Einstellung der Alarmparameter;
a.4 Definition der auszulösenden Maßnahmen bei Auftreten einer Nichtkonformität;
b. Ausgabephase, umfassend die folgenden Schritte:
b.1 Alarmaktivierung gemäß dem Verschreibungszeitpunkt;
b.2 Deaktivierung des Alarms durch Benutzer-Authentifizierung am Medikamentenspender (1) oder durch Betätigung der Multifunktionstaste (2);
b.3 nach Deaktivierung des Alarms wird das Rotationssystem (10.6) des Spendermoduls (10) durch die Leiterplatte aktiviert;
b.4 durch die Drehung der Teilereinheit (10.8) wird das Fach (10.2) mit den auszugebenden Medikationseinheiten im Wesentlichen mit der Öffnung (10.7) der Hülle (10.3) ausgerichtet;
b.5 die Medikationseinheiten fallen auf den Verteiler (3) ;
b.6 der Zählsensor erfasst den Durchgang der Medikationseinheiten zum Verteiler (3);
b.7 die Medikationseinheiten werden durch den Verteiler (3) zum Ausgabebecher (4) weitergeleitet;
b.8 der Gewichtssensor überprüft, ob das Gewicht der im Ausgabebecher (4) deponierten Medikationseinheiten dem vorgesehenen Gewicht entspricht;
b.9 ein Benutzer entnimmt den Ausgabebecher (4); und
b.10 der Ausgabebecher-Präsenzsensor erkennt die Entnahme des Ausgabebechers (4) aus dem Medikamentenspender (1).

## Revendications

1. Distributeur modulaire et automatique pour médicaments, comprenant:
- un châssis (9);
- un système de stockage comprenant:
- au moins un module distributeur amovible (10) comprenant une protection (10.3) entourant un séparateur de stockage (10.8) et comprenant une sortie (10.7) dans la partie inférieure; et
- un socle (5);
- un système de distribution qui comprend un routeur de médicaments (3) situé sous le module distributeur (10) et une tasse de réception (4) placée sous le routeur (3);
- le module distributeur (10) comprend un bras de guidage (10.4) situé sous le module distributeur (10);
- le socle (5):
- a un format sensiblement complémentaire au format des bras de guidage (10.4);
- comprend des glissières configurées pour recevoir les bras de guidage (10.4); et
- comprend un bras de guidage (10.4) destiné à fixer les mécanismes qui permettent d'installer et de désinstaller le module distributeur (10);
- le module distributeur (10) est fixé au socle (5) dans une position sensiblement verticale;
**caractérisé en ce que:**
- le module distributeur (10) comprend:
- un capteur de comptage qui vérifie si les doses de médicaments sont sorties du module distributeur (10) ;
- un capteur de pesée qui contrôle que les doses de médicaments ont été distribuées dans la tasse de réception (4);
- un capteur de présence de la tasse de réception qui confirme que la tasse de réception (4) a déjà été retirée ou est encore dans le distributeur de médicaments (1) ; et
- un moteur vibrant qui est activé si le capteur de comptage ne détecte pas le passage des doses de médicaments.

2. Distributeur selon la revendication précédente, **dans lequel** le capteur de comptage est sélectionné parmi un capteur infrarouge, un capteur à micro-ondes ou un capteur à ultrasons.

3. Distributeur selon l'une quelconque des revendications précédentes, **dans lequel** le bras de guidage (10.4) en forme en "L", dans lequel la paroi inférieure du bras de guidage (10.4) est insérée dans la rainure de la paroi inférieure du socle (5), la paroi latérale du bras de guidage (10.4) est insérée dans la paroi latérale du socle (5), et les deux parois indiquées ont une longueur sensiblement égale au rayon de la protection (10.3).

4. Distributeur selon l'une quelconque des revendications précédentes, **dans lequel** le module distributeur (10) comprend:
- a système de rotation (10.6) comprenant les composants de rotation du séparateur de stockage (10.8);
- un connecteur (10.5) reliant le système de gestion et de communication au système de rotation (10.6);
et le séparateur de stockage (10.8) comprenant au moins une division (10.1) qui sépare au moins un compartiment (10.2).

5. Distributeur selon la revendication précédente, **dans lequel** le compartiment (10.2) a une forme sensiblement trapézoïdale dont la largeur rétrécit à partir de la partie extérieure, la partie intérieure étant la paroi du centre du séparateur de stockage (10.8).

6. Distributeur selon la revendication précédente, **dans lequel** la taille du compartiment (10.2) peut être modifiée en ajustant la distance entre les divisions (10.1) en déplaçant une division (10.1) sur la paroi du centre du séparateur de stockage (10.8).

7. Distributeur selon l'une quelconque des revendications précédentes, **dans lequel** le module distributeur (10) a une forme sensiblement cylindrique.

8. Distributeur selon l'une quelconque des revendications précédentes, dans lequel la protection (10.3) a une forme sensiblement circulaire.

9. Distributeur selon l'une quelconque des revendications précédentes, **dans lequel** le châssis (9) comprend un capot (7), un bouton multifonction (2) et un panneau de commande (8) pour saisir les spécifications des caractéristiques de fonctionnement, à savoir les informations concernant l'utilisateur et la prescription correspondante, la fonctionnalité d'authentification de l'utilisateur, les paramètres d'alarme, les actions à déclencher dans le cas où une non-conformité serait décelée.

10. Distributeur selon l'une quelconque des revendications précédentes, **dans lequel** il comprend par ailleurs un composant électronique comprenant un circuit imprimé, un système de capteur et un système de gestion et de communication permettant la gestion électronique et informatique du distributeur.

11. Distributeur selon la revendication précédente, dans lequel la communication du distributeur de médicaments (1) avec les serveurs et les bases de données est configurée pour fonctionner dans un dispositif électronique fixe ou mobile.

12. Méthode de distribuer des médicaments à l'aide du distributeur selon les revendications 9-11, **caractérisé en ce qu'**elle comprend les étapes suivantes:
a. phase de paramétrisation comprenant les étapes suivantes:
a.1 introduire des informations concernant l'utilisateur et la prescription correspondante;
a.2 activer la fonctionnalité d'authentification de l'utilisateur;
a.3 établir les paramètres d'alarme;
a.4 définir les actions à déclencher dans le cas où une non-conformité serait décelée;
b. phase de distribution comprenant les étapes suivantes :
b.1 en fonction de l'heure de la prescription, l'alarme est activée;
b.2 l'alarme est désactivée par authentification de l'utilisateur sur le distributeur de médicaments (1) ou en appuyant sur le bouton multifonction (2);
b.3 après la désactivation de l'alarme, le système de rotation (10.6) du module distributeur (10) est activé par le circuit imprimé;
b.4 grâce à la rotation du séparateur de stockage (10.8), le compartiment (10.2) contenant les doses de médicaments à distribuer est sensiblement au même niveau que la sortie (10.7) de la protection (10.3);
b.5 la quantité de doses de médicaments à distribuer tombe dans le routeur (3);
b.6 le capteur de comptage détecte le passage des doses de médicaments vers le routeur (3);
b.7 les doses de médicaments sont dirigées vers la tasse de réception (4) par le routeur (3);
b.8 le capteur de pesée vérifie si le poids des doses de médicaments déposées dans la tasse de réception (4) correspond au poids des doses de médicaments qui devraient se trouver à l'intérieur de la tasse de réception (4);
b.9 un utilisateur récupère la tasse de réception (4); et
b.10 le capteur de présence de la tasse de réception vérifie que la tasse de réception a été retirée (4) du distributeur de médicaments (1).
